(19) Europäisches Patentamt
European Patent Office
Office européen des brevets

(11) Veröffentlichungsnummer : **0 465 421 A1**

(12) # EUROPÄISCHE PATENTANMELDUNG

(21) Anmeldenummer : **91810494.4**

(22) Anmeldetag : **25.06.91**

(51) Int. Cl.[5] : **C07D 209/50,** C08K 5/3417, C09B 57/04

(30) Priorität : **04.07.90 CH 2218/90**

(43) Veröffentlichungstag der Anmeldung : **08.01.92 Patentblatt 92/02**

(84) Benannte Vertragsstaaten : **BE DE FR GB IT**

(71) Anmelder : **CIBA-GEIGY AG**
**Klybeckstrasse 141**
**CH-4002 Basel (CH)**

(72) Erfinder : **von der Crone, Jost, Dr.**
**La Dey**
**CH-1732 Arconciel (CH)**

(54) **Isoindolinonverbindungen mit langkettigen Alkylthioethergruppen.**

(57) Isoindolinonverbindungen der Formeln

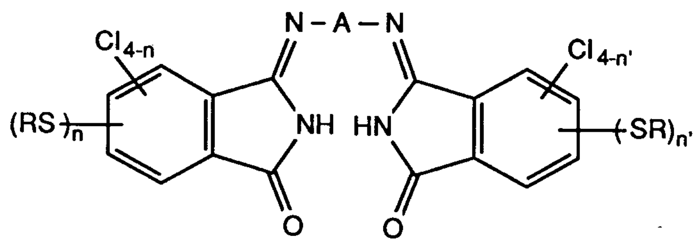

(I),

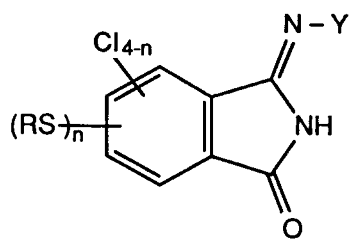

(II)

oder

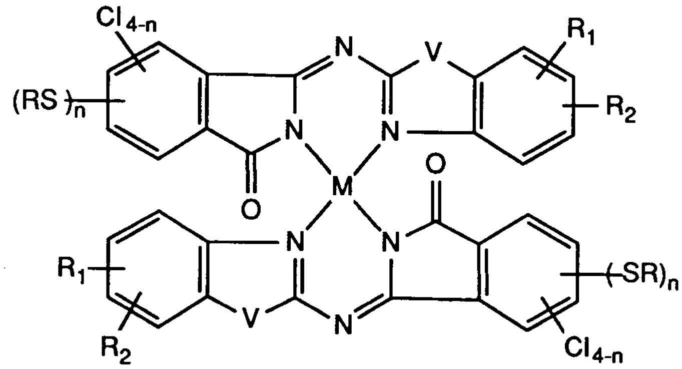

(III),

worin
R $C_8$-$C_{22}$-Alkyl bedeutet.
Für die weiteren Substituenten und Symbole wird auf Anspruch 1 verwiesen.

EP 0 465 421 A1

Jouve, 18, rue Saint-Denis, 75001 PARIS

Diese Isoindolinone eignen sich ausgezeichnet als Pigmente zum Färben von hochmolekularem organischem Material, insbesondere von Polyvinylchlorid und Polyolefinen.

Die vorliegende Erfindung betrifft Isoindolinonverbindungen enthaltend mindestens eine langkettige Alkylthioethergruppe und ihre Verwendung zum Färben von hochmolekularem organischem Material.

Isoindolinonverbindungen sind seit langer Zeit aus unzähligen Publikationen bekannt. Einige dieser Publikationen beschreiben u.a. auch Isoindolinonverbindungen, die Niederalkylthioethergruppen enthalten, so z.B. das US-Patent 3,867,404 mit Bisisoindolinonverbindungen, das US-Patent 3,884,955 und das CH-Patent 566 377 mit Monoisoindolinonverbindungen und das US-Patent 4,065,462 mit Isoindolinonmetallkomplexen. Ihre Eignung als Pigmente zum Färben von hochmolekularem organischem Material ist auch aus diesen Publikationen bekannt. Diese Pigmente genügen allerdings nicht immer den inzwischen für bestimmte Anwendungen stets höher gesetzten Erfordernissen, wie z.B. der guten Dispergierbarkeit bei der Massefärbung von Polyolefinen.

Es sind nun neue Isoindolinonverbindungen mit langkettigen Alkylthioethergruppen gefunden worden, die sehr gute Allgemeinbeständigkeiten zeigen und sich durch verbesserte Verarbeitungseigenschaften auszeichnen.

Die Erfindung betrifft demnach Isoindolinonverbindungen der Formeln

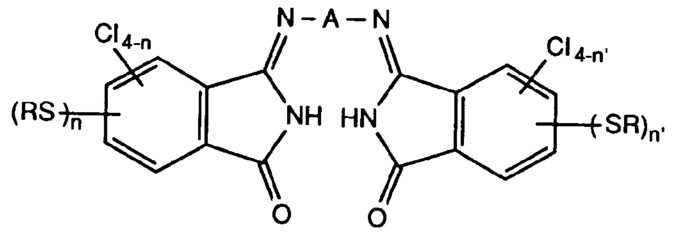

(I),

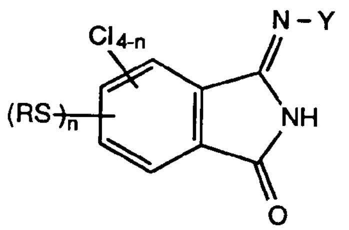

(II)

oder

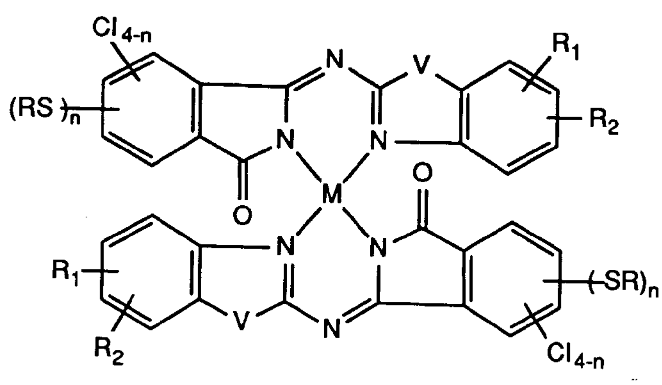

(III),

worin

A eine der Gruppen

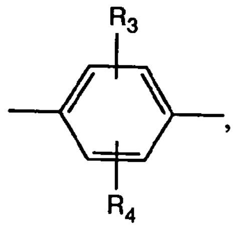
,
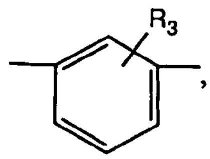
,
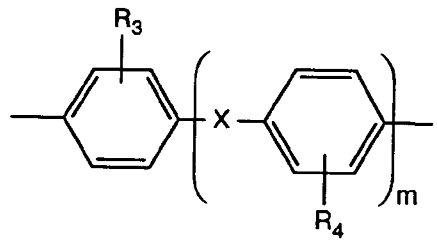

oder

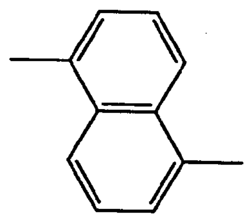

bedeutet, worin m die Zahlen 1, 2 oder 3, $R_3$ und $R_4$ unabhängig voneinander Wasserstoff, Halogen, $C_1$-$C_4$-Alkyl oder $C_1$-$C_4$-Alkoxy und X eine direkte Bindung, -O-, -S-, -NHCO-, -$OCH_2O$- oder -N=N- bedeuten,
n 1 oder 2 und n'Null, 1 oder 2 sind,
R $C_8$-$C_{22}$-Alkyl bedeutet,
$R_1$ und $R_2$ unabhängig voneinander Wasserstoff, Halogen, $C_1$-$C_4$-Alkyl oder $C_1$-$C_4$-Alkoxy sind,
Y eine der Gruppen

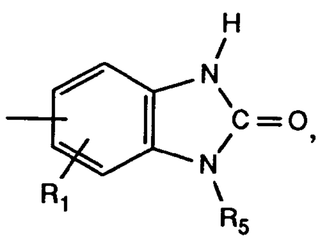
,
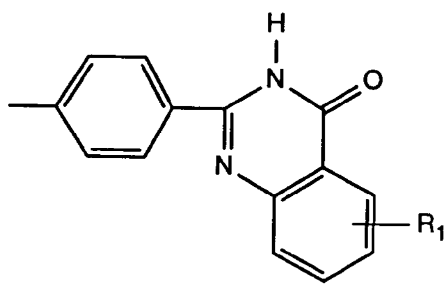
,

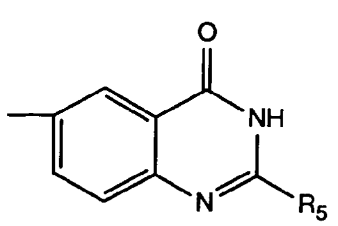
oder
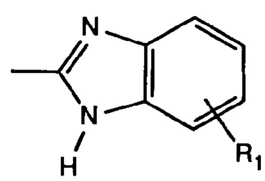

bedeudet, worin
$R_1$ die oben angegebene Bedeutung hat und $R_5$ Wasserstoff, $C_1$-$C_4$-Alkyl, unsubstituiertes oder durch Halogen, $C_1$-$C_4$-Alkyl, $C_1$-$C_4$-Alkoxy oder $H_2NCO$-($C_1$-$C_4$-Alkyl) substituiertes Phenyl ist,
M ein zweiwertiges Uebergangsmetall ist und
V -O-, -S- oder -NH- bedeutet.

Halogen bedeutet dabei z.B. Fluor, Brom, Jod und insbesondere Chlor.

Bedeuten etwaige Substituenten $C_1$-$C_4$-Alkyl, so handelt es sich um Methyl, Ethyl, n-Propyl, Isopropyl, n-Butyl, sec-Butyl oder tert.-Butyl.

Bedeuten etwaige Substituenten $C_1$-$C_4$-Alkoxy, so handelt es sich um Methoxy, Ethoxy, n-Propoxy, Isopropoxy, n-Butoxy, sec.-Butoxy oder tert.-Butoxy.

R als $C_8$-$C_{22}$-Alkyl bedeutet unverzweigtes oder verzweigtes Alkyl und ist z.B. n-Octyl, 1-, 2-, 3-, 4-, 5- oder 6-Methyl-heptyl, 1,1-Dimethyl-hexyl, 1,5-Dimethylhexyl, 1,1,3,3-Tetramethyl-butyl, n-Nonyl, 1,1-Dimethyl-heptyl, 1, 1,3,3-Tetramethyl-pentyl, n-Decyl, n-Dodecyl, 1,1,3,3,5,5-Hexamethyl-hexyl, Tridecyl, Tetradecyl, Pentadecyl, Hexadecyl, Octadecyl, Nonadecyl, Eicosyl, Heneicosyl oder Docosyl. Bevorzugt sind n-Octyl, 1-Methyl-heptyl, n-Nonyl, 1,1,3,3-Tetramethyl-pentyl, n-Dodecyl, 1,1,3,3,5,5-Hexamethyl-hexyl und n-Octadecyl.

M bedeutet als zweiwertiges Uebergangsmetall z.B. Cr, Mn, Fe, und insbesondere Co, Ni, Cu oder Zn.

Von besonderer Bedeutung sind Isoindolinonverbindungen der Formeln I, II und III, worin A eine der Gruppen

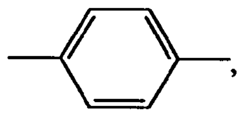 , 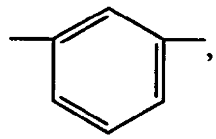 , 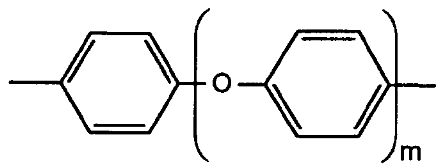

oder

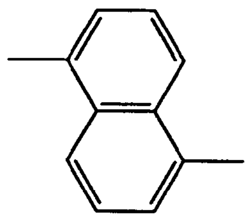

bedeudet,
$R_1$ und $R_2$ Wasserstoff sind,
Y eine Gruppe

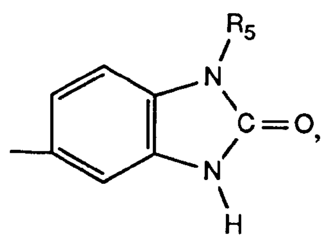

worin $R_5$ Wasserstoff oder Methyl ist, bedeutet,
M Co, Ni, Cu oder Zn ist,
V -NH- bedeutet,
und n, n'und R die oben angegebene Bedeutung haben, und darunter insbesondere die Verbindungen der Formel I.

Bevorzugt sind Verbindungen der Formel

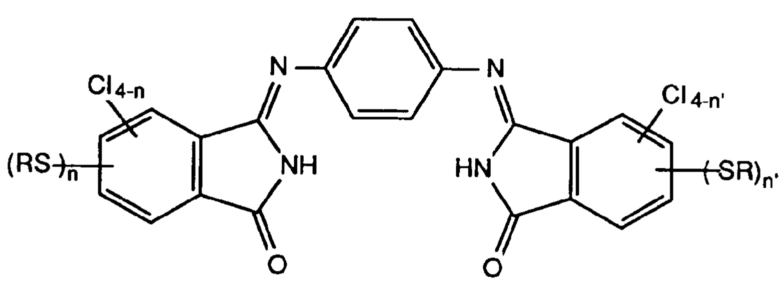 (IV),

worin n, n'und R die oben angegebene Bedeutung haben und insbesondere diejenigen der Formel

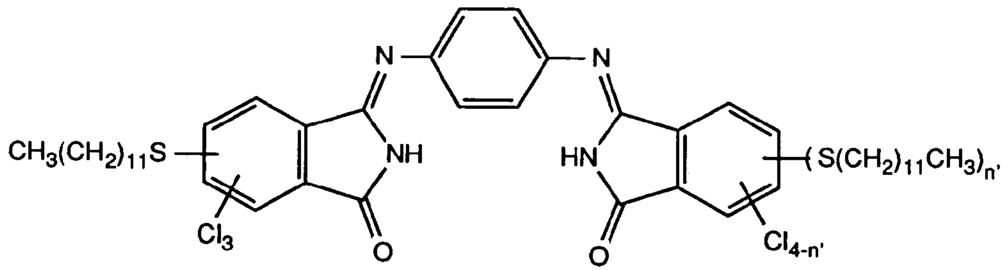 (V),

worin n' 0 oder 1 ist.

Die Isoindolinonverbindungen der Formeln I, II und III können in Analogie zu allgemein bekannten Methoden, etwa wie in den oben erwähnten Patentschriften beschrieben, hergestellt werden. Zweckmässig werden sie ausgehend von Verbindungen der Formel

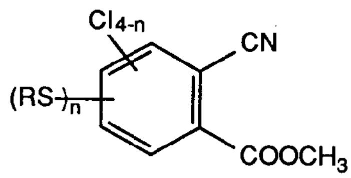 (VI)

durch eine erste Umsetzung mit methanolischer Na-Methylatlösung zu Verbindungen der Formel

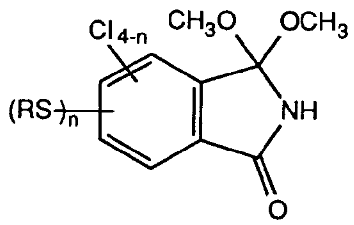 (VII)

(vgl. z.B. US-Patent 3,867,404) und anschliessende weitere Umsetzung

a) im Molverhätnis 2:1 mit einem Diamin der Formel

$$H_2N - A - NH_2 \quad (VIII)$$

zu Verbindungen der Formel I (vgl. z.B. ebenfalls US-Patent 3,867,404) oder

b) im Molverhätnis 1:1 mit einem Amin der Formel

$$H_2N - Y \quad (IX)$$

zu Verbindungen der Formel II (vgl. z.B. CH-Patent 566 377), hergestellt.

Bei der Umsetzung a) kann die Hälfte (in Mol) der Verbindung der Formel VII mit einer Verbindung der Formel

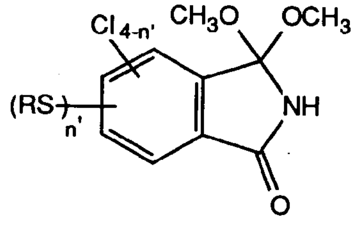

ersetzt werden.

Durch Umsetzung der Verbindungen der Formel II z.B. mit Co-, Ni-, Cu- oder Zn-Acetat im Molverhältnis 2:1 erhält man die Metallkomplexe der Formel III (vgl. z.B. US-Patent 4,065,462).

Die Verbindungen der Formel VI erhält man in Analogie zu allgemeinen bekannten Methoden, z.B. wie im US-Patent 3,884 955 beschrieben, durch Umsetzung von Tetra-chlor-2-cyanbenzoesäuremethylester beispielsweise mit einer Verbindung der Formel RS-K oder RS-Na. Es wird angenommen, dass das Chloratom

zuerst in 5-Stellung dann in 3-Stellung ausgetauscht wird.

Bei den Verbindungen der Formeln VIII und IX handelt es sich um bekannte und grösstenteils im Handel erhältliche Verbindungen.

Die erhaltenen erfindungsgemässen Isoindolinonverbindungen werden nach üblichen Methoden isoliert und getrocknet.

Die erfindungsgemässen Isoindolinonverbindungen eignen sich ausgezeichnet als Pigmente zum Färben von hochmolekularem organischem Material.

Hochmolekulare organische Materialien, die mit den erfindungsgemässen Pigmenten gefärbt bzw. pigmentiert werden können, sind z.B. Celluloseether und -ester, wie Ethylcellulose, Nitrocellulose, Celluloseacetat oder Cellulosebutyrat, natürliche Harze oder Kunstharze, wie Polymerisations- oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Besonders geeignet sind die erfindungsgemässen Pigmente zum Einfärben von Polyvinylchlorid und Polyolefinen, wie Polyethylen und Polypropylen, aber auch zum Pigmentieren von Lacken und Anstrichstoffen, insbesondere Automobillacken.

Die erfindungsgemässen Verbindungen der Formeln I, II und III können aufgrund ihrer ausgezeichneten Polyolefinverträglichkeit oft ohne weitere Konditionierung, wie mechanische Zerkleinerung oder Präparierung, zu deren Färbung direkt in Polyolefine eingearbeitet werden.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Pigmente als Toner oder in Form eines Präparates einzusetzen.

Bezogen auf das zu pigmentierende hochmolekulare organische Material kann man die erfindungsgemässen Pigmente in einer Menge von 0,01 bis 30 Gew.%, vorzugsweise von 0,1 bis 10 Gew. %, einsetzen.

Die Pigmentierung der hochmolekularen organischen Substanzen mit den erfindungsgemässen Pigmenten erfolgt beispielsweise derart, dass man das Pigment, gegebenenfalls in Form von Masterbatches, diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung des erfindungsgemässen Pigmentes in die Polymeren eingearbeitet werden. Zwecks Erzielung verschiedener Farbtöne ist es ferner möglich, den hochmolekularen organischen Stoffen neben dem erfindungsgemässen Pigment noch Füllstoffe bzw. andere farbgebende Bestandteile, wie Weiss-, Bunt- oder Schwarzpigmente, in beliebigen Mengen zuzufügen.

Zum Pigmentieren von lacken, Anstrichstoffen und Druckfarben werden die hochmolekularen organischen Materialien und die erfindungsgemässen Pigmente gegebenenfalls zusammen mit Zusatzstoffen, wie Füllmitteln, anderen Pigmenten, Siccativen oder Weich-machern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

In Färbungen, beispielsweise von Polyvinylchlorid oder Polyolefinen, zeichnen sich die erfindungsgemässen Pigmente durch gute allgemeine Pigmenteigenschaften, wie gute Dispergierbarkeit, hohe Farbstärke und Reinheit, sowie ausgezeichnete Migrations-, Hitze-, Licht- und Wetterbeständigkeit aus.

Die nachfolgenden Beispiele erläutern die Erfindung.

Beispiel 1 a): Herstellung des Zwischenproduktes

In eine Lösung von 59,8 g 3,4,5,6-Tetrachlor-2-cyanbenzoesäuremethylester und 200 ml Dimethylformamid wird eine Suspension von 30,4 g pulverisiertem Kaliumcarbonat, 52,8 ml 1-Dodecanthiol und 200 ml Dimethylformamid zugegeben. Es wird 5 Stunden bei Raumtemperatur gerührt und anschliessend auf 2 Liter Wasser aufgegossen. Das Reaktionsprodukt wird mit Methylenchlorid extrahiert, dann wird die Methylenchloridschicht getrocknet und eingedampft. Man erhält 95 g einer gelbbraunen Flüssigkeit, die hauptsächlich aus Dodecylmercapto-trichlor-2-cyanbenzoesäuremethylester besteht. Als Nebenprodukte sind die Tetrachlor- und Didodecylmercaptodichloroverbindungen enthalten.

b) Herstellung des Pigmentes

12,2 g des nach Beispiel 1a) hergestellten Reaktionsproduktes werden in 50 ml Toluol gelöst und mit 4,8 ml einer 30%-igen methanolischen Natriummethylat-Lösung versetzt. Man erwärmt auf 60°C und rührt 1 Stunde. Man lässt wieder auf Raumtemperatur abkühlen und gibt 1,35 g p-Phenylendiamin zu. Es entsteht eine tiefgefärbte Suspension, die 10 Stunden bei Raumtemperatur gerührt und anschliessend während 2 Stunden auf Rückflusstemperatur erhitzt wird. Nach Zugabe von 5 ml Eisessig entsteht eine dickflüssige orange Suspension, die mit 50 ml Toluol verdünnt wird. Man lässt auf Raumtemperatur abkühlen und filtriert das gebildete Pigment ab. Nach dem Waschen mit Toluol und Methanol und anschliessender Trocknung erhält man 7,9 g gelbes Pigment bestehend im wesentlichen aus der Verbindung der Formel

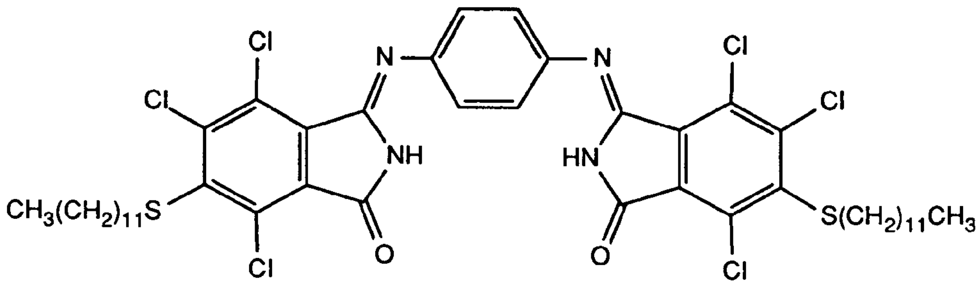

das sich sowohl für die Einfärbung von Polyvinylchlorid als auch für die Massefärbung von Polypropylen-Fasern in Gelbtönen mit hoher Lichtechtheit eignet.

Beispiel 2a): Herstellung des Zwischenproduktes

Eine Lösung von 43,7 g 1-Dodecanthiol und 38,9 ml einer 30%-igen methanolischen Natriummethylat-Lösung in 100 ml Dimethylformamid wird während 1 Stunde zu 29,9 g 3,4,5,6-Tetrachlor-2-cyanbenzoesäuremethylester in 100 ml Dimethylformamid zugetropft. Man rührt 3 Stunden bei Raumtemperatur und giesst dann auf 2 Liter Wasser auf. Nach Extraktion mit Methylenchlorid und Abdestillieren des Lösungsmittels erhält man 59,5 g einer gelblichen Flüssigkeit. Im Dünnschichtchromatogramm zeigt das Produkt eine praktisch einheitliche Zone, die dem Di-dodecylmercapto-dichlor-2-cyanbenzoesäuremethylester entspricht.

b) Herstellung des Liganden

21,5 g des nach Beispiel 2a) hergestellten Reaktionsproduktes werden in 100 ml Toluol gelöst und mit 6,26 ml einer 30%-igen methanolischen Natriummethylat-Lösung versetzt. Man rührt 15 Minuten und gibt dann 5,32 g 2-Aminobenzimidazol zu. Das tiefgefärbte Reaktionsgemisch wird 30 Minuten auf 60°C erwärmt und anschliessend mit 60 ml Methanol verdünnt. Man rührt 10 Stunden bei Raumtemperatur, erhitzt nochmals 1 Stunde auf 60°C und gibt dann 10 ml Eisessig zu. Man lässt auf Raumtemperatur abkühlen, fällt den Farbstoff mit 200 ml Methanol aus, filtriert ab und wäscht mit Methanol. Nach dem Trocknen erhält man 13,5 g gelbes Produkt bestehend im wesentlichen aus der Verbindung der Formel

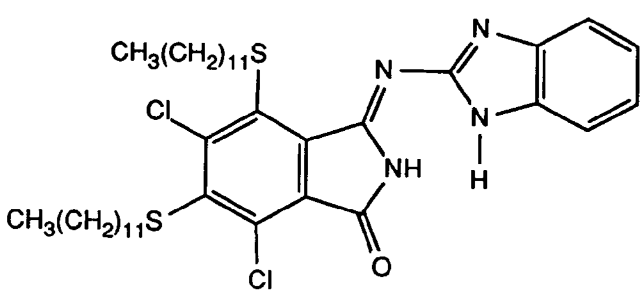

c) Herstellung des Metallkomplex-Pigmentes

3,7 g des gemäss Beispiel 2b) erhaltenen Produktes werden in 50 ml Ethylenglykolmonoethylether mit 0,67 g Kobaltacetat·$4H_2O$ auf Rückflusstemperatur erhitzt. Nach 2 Stunden lässt man abkühlen, filtriert den gebildeten Metallkomplex ab und wäscht mit Methanol nach. Man erhält 3,6 g eines orangen Produktes, das folgende Analyse zeigt:

Co gef. 3,94 % ; ber. 3,88 %.

Nach Einarbeitung in Polyethylen oder Polypropylen-Fasern erhält man rotstichig gelbe Einfärbungen mit hoher Hitzebeständigkeit.

Beispiele 3 und 4:

Nachfolgend sind 2 Pigmente beschrieben, die aus dem Liganden von Beispiel 2b) durch Umsetzung mit M-Acetat nach der Methode von Beispiel 2c) erhalten werden:

| Beispiel | M | Metallgehalt in % gef. | Metallgehalt in % ber. | Farbton in PE |
|---|---|---|---|---|
| 3 | Ni | 4,10 | 3,86 | gelb |
| 4 | Cu | 4,6 | 4,16 | gelb |

Beispiel 5:

6,4 g des nach Beispiel 1a) hergestellten Reakionsproduktes werden in 50 ml Toluol gelöst und mit 4,8 ml einer 30%-igen methanolischen Natriummethylat-Lösung versetzt. Man erwärmt 1 Stunde auf 60°C, lässt auf Raumtemperatur abkühlen und gibt eine Lösung von 3,9 g 3,4,5,6-Tetrachlor-2-cyanbenzoesäuremethylester und 2,5 ml 30%-iges methanolisches Natriummethylat in 50 ml Methanol zu. Nach 30 Minuten fügt man 1,35 g p-Phenylendiamin zu und rührt während 10 Stunden. Anschliessend wird 2 Stunden am Rückfluss erwärmt, 5 ml Eisessig zugegeben, nochmals 2 Stunden am Rückfluss erwärmt und auf Raumtemperatur abgekühlt. Nach Zugabe von 150 ml Methanol wird filtriert, mit Methanol und dann mit Wasser gewaschen und getrocknet. Man erhält 8,5 g rotstichig gelbes Produkt, das als Hauptkomponente das Pigment folgender Formel enthält

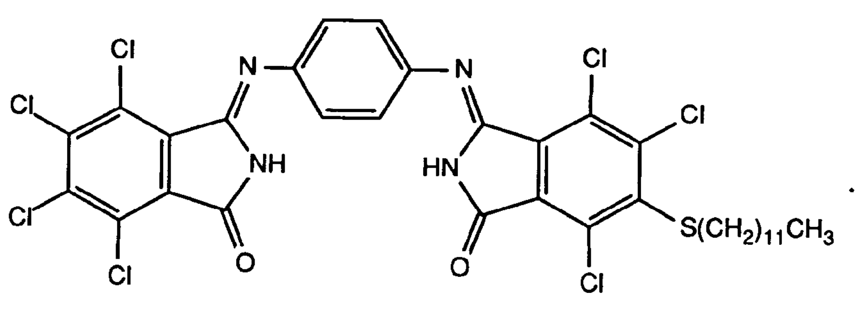

Polyvinylchlorid-Einfärbungen mit diesem Pigment zeichnen sich durch einen reinen, rotstichig gelben Farbton, hohe Farbstärke und hervorragende Lichtechtheit aus.

Beispiele 6-10:

In der nachfolgenden Tabelle sind Pigmente bestehend im wesentlichen aus Verbindungen der allgemeinen Formel

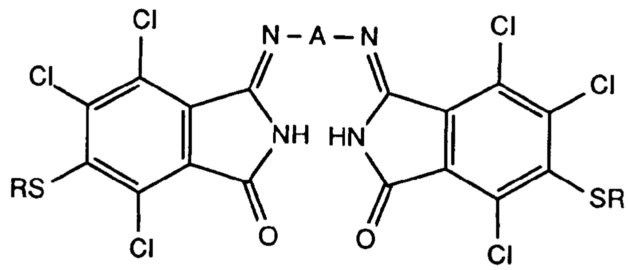

beschrieben, die beim Wiederholen des in Beispiel 1 beschriebenen Verfahrens durch Ersatz von 1-Dodecylthiol und p-Phenylendiamin mit äquimolaren Mengen von R-SH und $H_2N\text{-}A\text{-}NH_2$, wobei R und A die in der Tabelle angegebene Bedeutung haben, erhalten werden. In der letzten kolonne der Tabelle ist der jeweilige Farbton in PVC-Folien angegeben.

| Beispiel | R | A | Farbton in PVC |
|---|---|---|---|
| 6 | $CH_3(CH_2)_{11}$- | 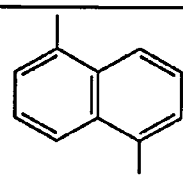 | Rot |
| 7 | $CH_3(CH_2)_{11}$- | 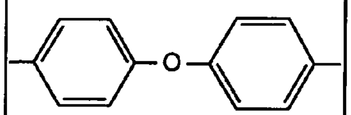 | Gelb |
| 8 | $CH_3(CH_2)_{11}$- | 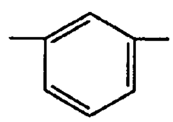 | Grünstichig Gelb |
| 9 | $(CH_3)_3CCH_2C(CH_3)_2CH_2C(CH_3)_2$- | 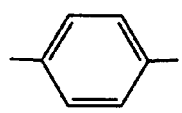 | Gelb |
| 10 | $CH_3(CH_2)_{17}$- | 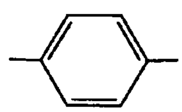 | Gelb |
| 11 | $C_2H_5C(CH_3)_2C(CH_3)_2$- | 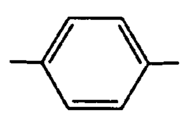 | Gelb |
| 12 | $CH_3(CH_2)_7$- | 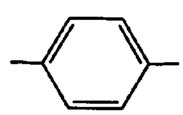 | Gelb |

Beispiel 13:

40 mg des gemäss Beispiel 1b) erhaltenen Isoindolinonpigmentes werden mit 7,3 ml Dioctylphthalat und 13,3 g eines stabilisierten Polyvinylchlorids vom Typ ®LONZA E-722 in einem Becherglas mit einem Glasstab gut vermischt. Das erhaltene Gemisch wird dann auf einem Walzenstuhl während 5 Minuten bei 160°C zu einer dünnen Folie verarbeitet. Die so erzeugte PVC-Folie weist eine farbstarke gelbe Färbung hoher Reinheit und sehr guter Lichtbeständigkeit auf. Die Dispergierbarkeit des Pigmentes ist ausgezeichnet.

Beispiel 14:

Eine Mischung von 1,0 g des nach Beispiel 1b) erhaltenen Isoindolinonpigmentes, 1,0 g Antioxidans ®IRGANOX 1010 (CIBA-GEIGY AG) und 1000 g Polyethylen HD Granulat (®VESTOLEN A60-16, HUELS) wird während 15 Minuten in einer 3 1-Glasflasche auf einer Rollbank vorgemischt. Danach wird die Mischung zweimal durch einen Einwellenextruder extrudiert. Das so erhaltene Granulat wird dann auf einer Spritzgussmaschine (®Allround Aarburg 200) bei 250°C mit einer Verweilzeit von 5 Minuten zu Platten verspritzt. Die so erhaltenen Platten weisen gleichmässig farbstarke gelbe Färbungen hoher Reinheit und ausgezeichneter Lichtbeständigkeit auf.

Beispiel 15:

1000 g Polypropylengranulat (®DAPLEN PT-55, Chemie LINZ) und 1,0 g des nach Beispiel 2c) erhaltenen Isoindolinonpigmentes werden in einer 3 1-Flasche während 15 Minuten auf dem Rollbock gemischt. Danach wird die Mischung zweimal durch einen Einwellenextruder extrudiert und anschliessend granuliert. Das so erhaltene Granulat wird bei 280-285°C nach dem Schmelzspinnverfahren versponnen. Die so gefärbten gelben Fasern weisen eine sehr gute Lichtbeständigkeit und ausgezeichnete textile Echtheiten, wie Reib- und Nassechtheiten gegenüber Wasch- und Lösungsmitteln, auf. Die Hitzebeständigkeit des Pigments während des Spinnprozesses bei 285°C ist ausgezeichnet.

Beispiel 16:

Verfährt man analog wie in Beispiel 14 beschrieben, verwendet aber neben dem Buntpigment 10 g Titandioxid ®KRONOS RN-57-P (KRONOS Titan GmbH), so erhält man gelbe Pressplatten mit ebensoguten Hitzebeständigkeiten. Die zwischen 200 und 280°C gespritzten Pressplatten weisen nach dem Erkalten keine Farbabweichungen auf.

**Patentansprüche**

1. Isoindolinonverbindungen der Formeln

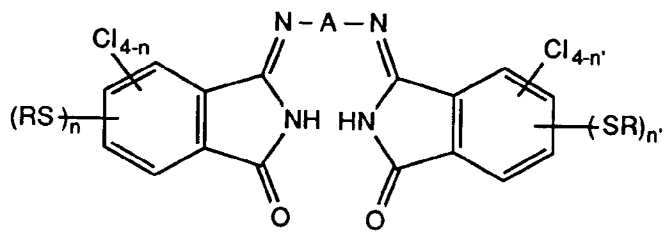

(I),

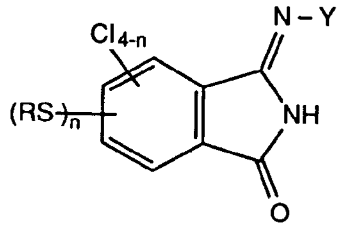

(II)

oder

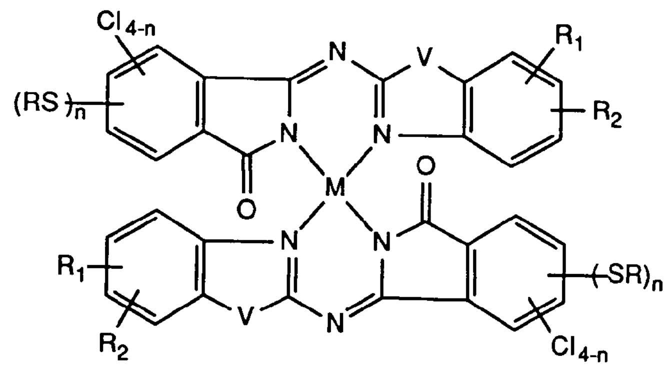

(III),

worin
A eine der Gruppen

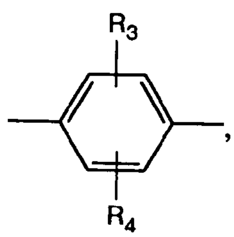

,

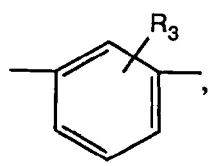

,

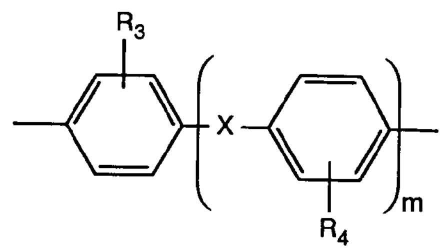

oder

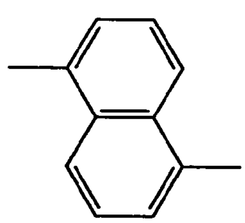

bedeutet, worin m die Zahlen 1, 2 oder 3, $R_3$ und $R_4$ unabhängig voneinander Wasserstoff, Halogen, $C_1$-$C_4$-Alkyl oder $C_1$-$C_4$-Alkoxy und X eine direkte Bindung, -O-, -S-, -NHCO-, $-OCH_2O-$ oder -N=N- bedeuten, n 1 oder 2 und n'Null, 1 oder 2 sind,
R $C_8$-$C_{22}$-Alkyl bedeutet,
$R_1$ und $R_2$ unabhängig voneinander Wasserstoff, Halogen, $C_1$-$C_4$-Alkyl oder $C_1$-$C_4$- Alkoxy sind,
Y eine der Gruppen

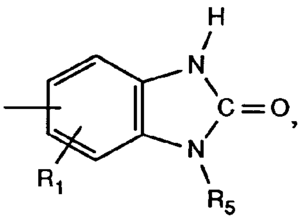,
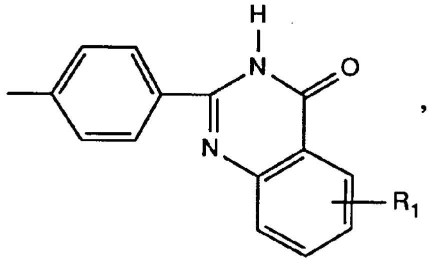,
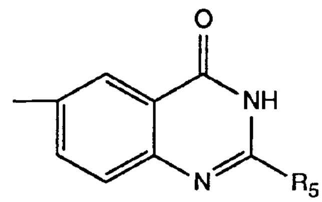

oder 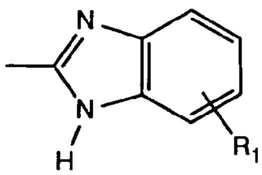

bedeudet, worin $R_1$ die oben angegebene Bedeutung hat und $R_5$ Wasserstoff, $C_1$-$C_4$-Alkyl, unsubstituiertes oder durch Halogen, $C_1$-$C_4$-Alkyl, $C_1$-$C_4$-Alkoxy oder $H_2NCO$-($C_1$-$C_4$-Alkyl) substituiertes Phenyl ist,
M ein zweiwertiges Uebergangsmetall ist und
V -O-, -S- oder -NH- bedeutet.

**2.** Isoindolinonverbindungen der Formeln I, II und III gemäss Anspruch 1, worin
A eine der Gruppen

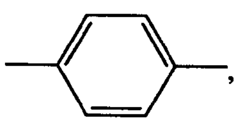,
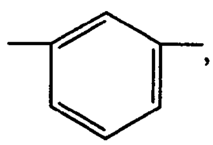,
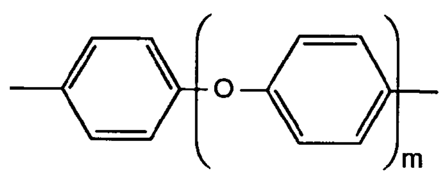

oder

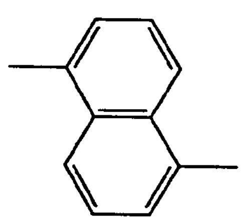

bedeutet, $R_1$ und $R_2$ Wasserstoff sind,
Y eine Gruppe

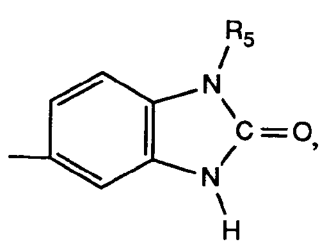,

worin $R_5$ Wasserstoff oder Methyl ist, bedeutet,
M Co, Ni, Cu oder Zn ist und
V -NH- bedeutet.

**3.** Isoindolinonverbindungen der Formel I gemäss Anspruch 1.

**4.** Isoindolinonverbindungen gemäss Anspruch 1 der Formel

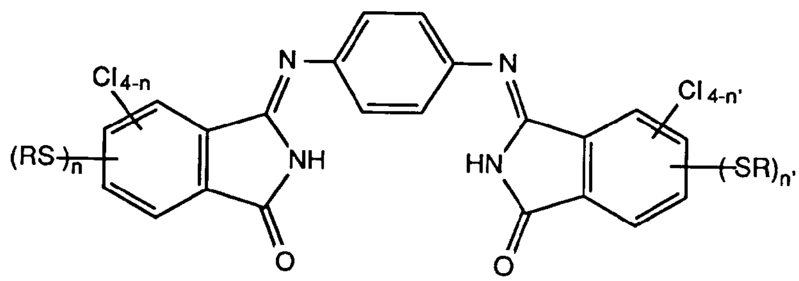

(IV),

worin n, n'und R die in Anspruch 1 angegebene Bedeutung haben.

**5.** Isoindolinonverbindungen gemäss Anspruch 1 der Formel

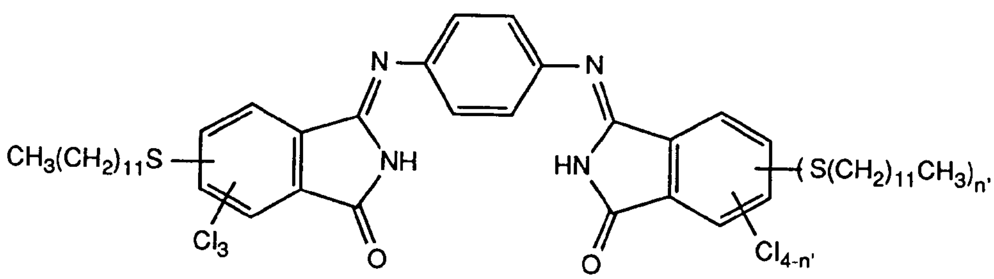

(V),

worin n'0 oder 1 ist.

**6.** Hochmolekulares organisches Material enthaltend als Pigment mindestens eine der Isoindolinonverbindungen der Formeln I, II und/oder III gemäss Anspruch 1.

Europäisches Patentamt

# EUROPÄISCHER RECHERCHENBERICHT

Nummer der Anmeldung

EP 91 81 0494

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | US-A-3867404 (J. V.D.CRONE ET AL.)<br>* Ansprüche 1, 2, 4, 10 * | 1-4 | C07D209/50<br>C08K5/3417<br>C09B57/04 |
| Y | EP-A-0062615 (CIBA-GEIGY AG)<br>* Anspruch 1; Beispiele 13, 16 * | 1-4 | |
| A | US-A-4070367 (E. MODEL)<br>* Zusammenfassung; Anspruch 1 * | 1, 2, 6 | |
| A<br><br>D | US-A-4052410 (J. V.D.CRONE ET AL.)<br>* Zusammenfassung; Anspruch 1 *<br>& CH-A-566377 | 1, 2, 6 | |
| A,D | US-A-4065462 (C. FREY ET AL.)<br>* Zusammenfassung; Anspruch 1 * | 1, 2, 6 | |
| A<br><br>D | DE-A-2301863 (CIBA-GEIGY AG)<br>* Seite 16 unten, Formel *<br>& US-A-3884955 | 1 | |
| | | | RECHERCHIERTE SACHGEBIETE (Int. Cl.5 ) |
| | | | C07D209/00<br>C08K5/00<br>C09B57/00 |

Der vorliegende Recherchenbericht wurde für alle Patentansprüche erstellt

| Recherchenort | Abschlußdatum der Recherche | Prüfer |
|---|---|---|
| BERLIN | 16 OKTOBER 1991 | HASS C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)